# EUROPEAN PATENT APPLICATION

(11) **EP 1 318 398 A1**
(43) Date of publication of application: **11.06.2003**
(21) Application number: 01128418.9
(22) Date of filing: 04.12.2001
(51) Int. Cl.: G01N 33/18

(54) **System and method for health monitoring of aquatic species**

(71) Applicant: GSF-Forschungszentrum für Umwelt und Gesundheit GmbH, 85764 Oberschleissheim (DE)
(72) Inventor: Brielmeier, Markus, 85748 Garching (DE); Knapik, Elzbieta, 80803 München (DE); Schmidt, Jörg, 80538 München (DE)
(74) Representative: Reinhard - Skuhra - Weise & Partner

(57) **Abstract**

The present invention provides a system and a method for health monitoring of aquatic species in aquacultures comprising a plurality of containments (4a, 4b, 4c, 4d, 4e, 4f) for aquatic species, wherein a sample of used water from at least one containment is supplied from at least one sample point (A, B, C,...,N) to at least one sentinel containment housing sentinel aquatic species as bio-indicators for the detection of infectious particles and/or chemical factors and/or physical factors in said supplied water sample.

## Description

### BACKGROUND OF THE INVENTION

The present invention refers to a system and a method for health monitoring of aquatic species in aquacultures for detecting detrimental factors, e. g. infectious particles, within a certain containment. The present invention relates generally to all aquatic organisms, including Rana and Xenopus species. Hereinafter they are all referred to by the term "fish".

In the field of laboratory animal science, pet fish and seafood production, aquaculture systems are used to breed and maintain aquatic species. Aquaculture systems according to the state of the art consist of a set of aquaria, a life support system and a water source.

Bulk holding of conventionally kept as well as of a specific-pathogen-free (SPF) laboratory fish gains rapidly increasing importance in basic and applied biomedical research. Breeding and housing of fish requires a defined environment and climate. Also, pathogenic micro-organisms, such as viruses, bacteria and parasites as well as chemicals and physical factors may dramatically affect fish health in colonies of any size and habitat and exert deleterious effects. Hygienic standards for fish containments have not yet been established. Health monitoring implies regular analysis of the water and its effects on fish health as it relates to infections with viruses, bacteria and parasites. Health monitoring also implies regular analysis of the water and its effects on fish health as it relates to chemicals or to physical factors. In particular, chemical monitoring implies regular analysis of the chemical contents, in particular chemicals and/or heavy metals and/or radioactive substances, and physical monitoring implies regular analysis of the radioactivity of the water and of the fish.

Currently, there is no standard established to test or monitor potential contamination with heavy metals or organic compounds. In many scientific or commercial fish facilities, random sampling for potential biological contamination is difficult to conduct due to the large number of tanks in the total volume of water. Therefore, most aquacultures are not monitored at all. But there is a high danger and a lot of sources for potential contamination. The aquaculture system is supplied with sterile water, salts and fish food. All fish inhabiting the system are either born within the system or imported as sterilized embryos before hatching, however, in live embryos, the efficacy of sterilization is difficult to assess.

Also contact of scientists and fish care-takers with wild fish or pet fish represents constant hazard of infections.

Further due to optimal living conditions, aquacultures are highly attractive to insects for deposition of eggs and subsequent development of larvae.

Furthermore, there is a high risk of infection of fish with bacteria and viruses from the environment. In cases of infection, the re-circulating system, for example, greatly facilitates the transmission of pathogens and spreading of diseases from single aquaria to the entire system.

Similar hazards result from chemicals and physical contamination through external system supplies, tubing and electromechanical parts. Especially the flow-through systems are not free of risk of contamination or infection. This is mainly due to common working space, handling and poor sterilization practices.

### SUMMARY OF THE INVENTION

Accordingly, it is an object of the present invention to provide a system and a method for detecting infectious particles in the water upstream certain containments.

This object is achieved with a system having the features of claim 1 and a method having the features of claim 15.

The invention provides a system for health monitoring of aquatic species in aquacultures comprising a plurality of containments for aquatic species, wherein a sample of used water from at least one containment is supplied from at least one sample point to at least one sentinel containment housing sentinel aquatic species as bio-indicators for the detection of infectious particles in said supplied water sample.

The system of the present invention provides a system for the detection of microbial infections and pathogens, for chemicals and for physical, e.g. radiological, factors in the aquatic environment of fish, which are kept in open and confined freshwater and marine containments as well as in the open sea, to improve health monitoring of fish. Therefore, water taken from a given aquatic area or containment is examined by the monitoring system using sentinel aquatic species as bio-indicators.

In a preferred embodiment, the system comprises at least one quality monitoring device for an additional examination of the used water supplied from the at least one sample point.

The containments are preferably aquaria, tanks, basins, pools, partitions of creeks, rivers or lakes and such like.

The system is in a preferred embodiment a re-circulating system.

In a further preferred embodiment, the system comprises at least one fresh water reservoir for supplying fresh water via water supply pipes to the containments.

Preferably, the system comprises water pumps providing a constant pressure in the system and water renewal in said containments.

The system preferably comprises a filtration system.

In a preferred embodiment, said filtration system comprises at least a large pore-filter unit, a particulate-filter unit, a fine-filter unit, a bio-filter unit, an activated carbon-filter unit and/or a UV-sterilization unit.

In a further preferred embodiment, the system comprises collecting pipes for collecting and supplying the used water to said particulate-filter unit, said bio-filter unit and said activated carbon-filter unit, being placed behind each other in downstream direction.

The system preferably comprises a pump reservoir to which the used water is supplied via said particulate-filter unit, said bio-filter unit and said activated carbon-filter unit and to which tap water is supplied via a reverse osmosis unit.

In another preferred embodiment, the UV-sterilization unit is placed between the pump reservoir and the fresh water reservoir.

Preferably, the fine-filter unit is placed between the UV-sterilization unit and the fresh water reservoir.

In a preferred embodiment, sample points are placed for fresh water sampling, reservoir water sampling, sentinel containment sampling, exit water sampling, pipe sampling, filter sampling, fresh tap water sampling, reverse osmosis unit sampling, pump reservoir sampling, pump sampling, UV-sterilization unit sampling and/or fine-filter unit sampling.

In an alternative preferred embodiment, the aquatic species are fish and the sentinel aquatic species are fish, which are highly susceptible for fish pathogens.

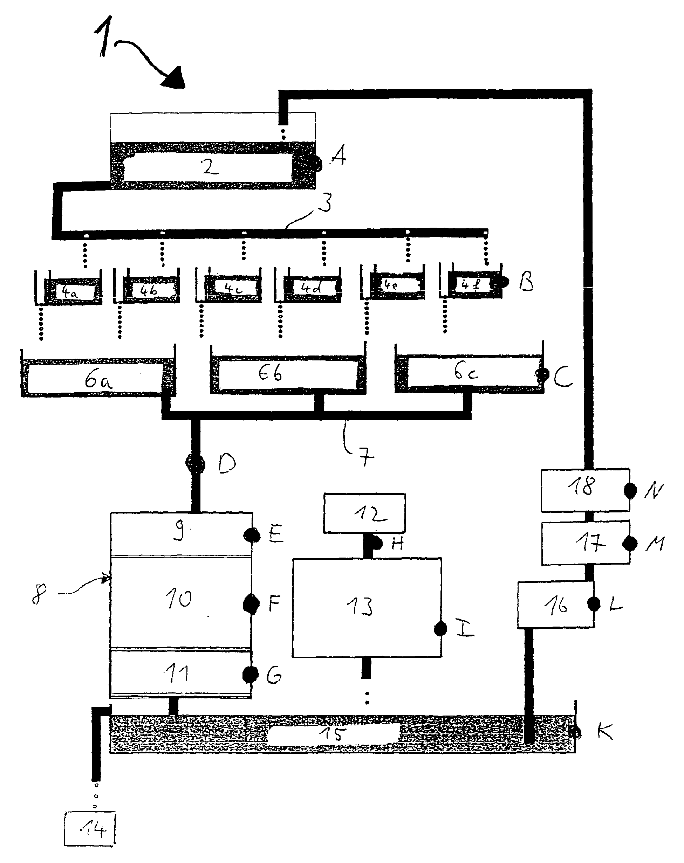

### BRIEF DESCRIPTION OF THE DRAWING

The Figure shows a preferred embodiment of the system for health monitoring of aquatic species in aquacultures to explain essential features of the present invention.

### DETAILED DESCRIPTION OF THE PREFERRED EMBODIMENT

Aquaculture systems are used, as mentioned above, to breed and maintain fish, Rana and Xenopus species, for example in research laboratories, in which, for example, zebrafish (danio rerio) is mainly used. Typically, three types of husbandry systems are used in these research laboratories.

In a static system, the water is neither flowing nor filtered. This system is mainly used for short periods of time for acute toxicity tests or other short experiments, and fish are not usually maintained for more than one week.

In the flow-through system, water enters the aquaculture system from a source, flows through the aquaria one time and exits as spillage. Variations of the flow-through system includes constant flow-through, timed flow-through, reservoir-timed flow-through and balanced water delivery systems.

More commonly used are the re-circulating systems, of which a preferred embodiment is shown in the Figure. Water enters the aquacultural system from a source and circulates through aquaria as a closed system. In this type of aquaculture, a small percentage of water is spilled and the same percentage is renewed. Spilled water contains preferentially the suspensions and sediments of the excretions of the fish.

Water is of far greater importance to fish as an environmental medium than air is to terrestrial animals. Therefore, the water quality is the primary requirement for a healthy system. The best results are obtained when reverse osmosis water from a reverse osmosis unit 13 supplied with tap water is used and subsequently adjusted for hardness and salinity to assure osmo-regulation. This method assures a biologically clean water without microbiological contamination and no chemical contamination coming from daily fluctuation of city/or source water. Furthermore, this method assures reproducibly consistent water quality. According to the present embodiment in the Figure, tap water from a tap water unit 12 is supplied to the reverse osmosis unit 13, for example via pipes.

A life support system supports regulated water flow through aquaria and maintains the system water quality. According to the described preferred embodiment, the life support system comprises water pumps 16, responsible for constant pressure in the system and water renewal in the tanks, a UV-sterilization unit 17 against viral or bacterial contamination, a fine-filter unit 18 to further purify the water and a filtration system 8 comprising a particulate-filter unit or large-pore filter unit 9 for a filtration of lagers debris and fish waste, a bio-filter unit 10 populated by defined ammonium- and nitrite-processing bacteria and an activated carbon-filter unit 11 to reduce organic compound contamination.

Typical systems contain four types of aquaria. Small containments with reduced water flow for rearing baby fish, large aquaria for rising families, small aquaria with normal water flow for keeping small families and specially identified fish, and single-fish containments for the long-term keeping of a single fish. Normally all aquaria are connected by a system of pipes allowing water entry to the aquarium from the clean water reservoir 2 and water exit towards the life support system.

Starting from the clean water reservoir 2, clean water is flowing by forces of gravity or by pumps through a system of water supply pipes 3 to a whole aquaculture system. As shown in the Figure, the aquaculture system of the preferred embodiment comprises a plurality of fish tanks 4a, 4b, 4c, ... 4f. It is clear to a person skilled in the art that different kinds of assemblies of single containments to a whole aquaculture system are possible. The water supply pipes 3 are distributing water such that the clean system water is flowing only once through a single aquarium, and preferably 5-10% of the system water is drained by overflow to the waste by a waste water unit 14. This flow is regulated by the thickness of the pipes and by a system of valves. The used system water exits the aquarium by overflow. The used water is collected by exit water tanks 6a, 6b, 6c and supplied to the filtration system 8 via water collecting pipes 7.

The set of main collecting pipes 7 delivers the used water to preferably large debris filter mats 9, bio-filter mats 10 harboring ammonium-processing bacteria and to a carbon-filter unit 11. The processed system water is collected into a pump reservoir 15.

At this point, new reverse osmosis water is added in a volume preferably 5-10% to the 95-90% of the total system water per day.

Pumps 16 are pumping renewed system water through the UV-sterilization unit 17 and subsequently through the fine-filter unit 18 towards the clean system water reservoir 2.

The quality of the water and effectiveness of the filtration system 8, 17, 18 are of greatest importance. A daily measurement of water salinity, pH-rate, temperature, air temperature and humidity should be performed daily. On a weekly basis, the level of ammonium, nitrite and nitrate should be measured. In well oxygenated systems, the level of oxygen and carbon dioxide should be measured periodically.

According to the embodiment of the present invention, water examination for health monitoring of fish can be executed by installation of a sampling device at every point of interest, for example A, B, C, ... N in a given aquaculture system. Depending on the arrangement of the aquaria and the system, the used water of the system 1 can be examined by single tank sampling B, row sampling D, partition sampling C, rack sampling, system sampling or such like to obtain information about the excretion of the fish into the water.

Depending on the arrangement of the life support system and the water flow in the system 1, the water supporting the aquaculture system can be sampled or examined by particulate-filter sampling E, bio-filter sampling F, carbon-filter sampling G, used water reservoir sampling K, pump sampling L, UV-sterilization sampling M and/or fine-filter sampling N to obtain information about the quality of the life support system.

Depending on the water flow, the aquaculture system 1 can be sampled or examined by fresh water sampling or water reservoir sampling A, main pipe sampling, UV sterilization unit sampling M and fine-filter sampling N to obtain information about the biological, chemical and physical quality of the water.

In the re-circulating system 1, as shown in the Figure, or in a flow-through system, preferably sentinel fish are used for microbiological, chemical and physical monitoring of the system 1 and of fish health. Different kinds of fish with highest susceptibility to biological, chemical or physical agents can be used to optimize the hazard/susceptibility ratio at an optimal rate. The sentinel containment housing sentinel fish as bio-indicators for the detection of infectious particles in said containment is called a Device for Improved Health Monitoring (DIHM). This device facilitates the monitoring of water quality and the monitoring of fish health. The DIHMs are installed at all points of interest in the system 1. Depending on the arrangement of the aquaria in the system, the DIHMs can be installed for tank monitoring B, row monitoring D, partition monitoring C, system monitoring to obtain information about the biological status of the system 1 upstream of the sampling point A, B, C, ... N.

Analogous to the aforementioned, DIHMs can be installed depending on the arrangement of the life support system for water pumps monitoring L, large pore-filter monitoring E, bio-filter monitoring F, carbon-filter monitoring G, UV-sterilization unit monitoring M, fine-filter monitoring N or such like to obtain information about the quality of the life support system.

Also analogous to the aforementioned, the DIHMs can be installed depending on the water flow in the system for fresh water reservoir monitoring A, used water reservoir monitoring K, main pipes monitoring, UV-sterilization unit monitoring M, fine-filter monitoring N, reverse osmosis unit monitoring I, tap water monitoring H to obtain information about the chemical and physical quality of the water.

Preferably, sentinel fish are constantly kept in DIHMs for a period of three months or less in the water examined in the different sampling points, A, B, C, ... N. Thereafter, the sentinel fish are taken and investigated as representatives of the fish kept upstream of the sampling point, respectively, for viral, bacterial and parasitical infections, for chemicals and physical agents and for contamination with radioactive nucleotides. Sentinel fish can also be subjected to regularly repeated pathological dissections to detect morphological alterations or signs of diseases, e.g. inflammations, necrosis, tumours, etc.. Sentinel fish can also be used for immunological analyses for infections with microbial agents.

The data obtained from the sentinel fish indicate the health status of all fish located upstream, whose water was monitored by the sentinel fish. The number of sentinel fish used in a particular aquaculture should be large enough to be statistically representative for the number of fish kept or monitored upstream of the sample point.

Aquaculture water can be tested at any interesting sampling point A, B, C, ... N for biological, chemical or physical constituents, which represent important factors for the quality of the water in the unit and for the functional status of each constituent of the life support system in experimental set-ups for marine containments as well as in the open sea.

The DIHM is designed to capture clean water or water with left-over food or fish excrements from different points of the aquaculture system, thereby examining aliquots of water from upstream compartments. The sampled or examined water is directed and delivered continuously or in intervals or fractions to an individual DIHM-tank, which houses sensitive and susceptible sentinel fish. These sentinel fish are continuously observed and investigated in regular intervals for the development of latent or overt signs of impaired health. By continuous use of DIHMs, sentinel fish are exclusively exposed to water from upstream compartments. An unaffected or impaired health status of sentinel fish indicates the absence or presence of pathogenic factors or organisms upstream of the sampling point.

Hence, this method represents online biological, chemical and physical monitoring of all fish housed in an aquaculture system and greatly increases the frequency of detection of pathogens, which are transported via the water to the sentinel fish.

A DIHM comprises at least a sampling tube introduced into either tanks or water compartments which carry a part or the total amount of water coming from all or selected fish housed upstream of the DIHM. Further, the DIHM comprises a sentinel tank housing sentinel fish which are preferably highly susceptible for fish pathogens. The sampled water containing potential pathogens from upstream fish is flowing into this individual tank by forces of gravity or by pressure produced by a pump. Sentinel fish in the DIHM are exposed to the total examined water and serve as bio-indicators for infectious pathogens which are excreted by experimental fish located upstream of the sentinel fish as well as for chemical or physical factors affecting fish health, as described above.

The DIHM further comprises a waste water tube from the sentinel containment which transports the water from the sentinel tank to the system waste water exit 14.

To monitor individually defined sets of fish harbouring compartments in aquaculture systems, the water from the compartments can be examined by system sampling, partition sampling, tank sampling and/or life support system sampling.

System sampling is executed at any defined point within the aquaculture system. Sampling water from the system monitors water from all fish contained in the system. Sampling water from the system requires a number of DIHMs equal to the number of desired defined sampling points.

Partition sampling is executed at any point within a defined partition of the aquaculture system. Sampling water from a partition of the system monitors water from all fish contained in the partition. Sampling water from partitions of the system requires a number of DIHMs equal to the number of desired partitions.

Tank sampling is executed at the level of a single tank. Sampling water from a tank monitors water from all fish contained in the tank. Sampling water from tanks requires a number of DIHMs equal to the number of tanks to be monitored.

Life support system sampling is executed at any defined point of the life support system. Sampling water from a defined point of the life support system monitors parts of the life support system. Sampling water from a defined point of the life support system requires a number of DIHMs equal to the number of defined sampling points.

Hence, the present invention provides a system and a method for health monitoring of aquatic species in aquacultures to detect infectious particles in certain containments. Thus, a fast reaction is possible to eliminate the source of the infectious particles.

DIHMs can be built into pre-existing aquaculture systems or stand-alone systems by fitting the appropriate sampling points to the system and monitor the aquaculture system on a system level, rack-level, partition level, tank level, life support system level or any other level according to the user's decision.

Furthermore, DIHMs can be built into stand-alone aquatic housing systems by the manufacturer to monitor fish health on a rack level, side level, row or partition level or on a single-tank level. In each case, water reaches the sentinel tank from the sampling point. The water of the respective upstream compartment is used as influx into the sentinel tank. The efflux water from the sentinel tank, which operates on the principle of flow-through system, is transported to the system waste water exit.

Additional sampling points may be installed at any point according to the user's request.

Finally, it should be mentioned that fresh water or marine systems are used to breed, maintain and produce ornamental pet fish and commercial fish for human consumption. Typically, two types of environments are used.

Sweetwater fish are kept in containments, e.g. aquaria, fish tanks, basins, pools, in partitions of creeks or rivers or in lakes. Aquaria, fish tanks, basins and pools are generally re-circulating systems into which water enters from a source and circulates as a closed system. A small percentage of water is spilled and the same percentage is renewed.

Partitions of creeks, rivers or lakes are flow-through systems. In the flow-through system, water enters the system from a source, flows through the system one time and exits as waste water or spillage. Marine systems are used to breed, keep and produce commercial fish in large quantities, whereby trout and Atlantic salmon (salmo salar) is used for the most part. The open sea environment is generally a fjord-like geographical situation with a connection to the open sea. In this system, the water is renewed by tidal convections and the water quality increases in a sea-bound gradient.

In the drawing and the specification, there has been set forth a preferred embodiment of the invention and, although specific terms are employed, the terms are used in a generic and descriptive sense only and not for the purpose of limitation, the scope of the invention being set forth in the following claims.

### List of Reference Signs

- 1: system
- 2: clean water reservoir
- 3: water supply pipes
- 4a, 4b, 4c, ... 4f: fish tank
- 6a, 6b, 6c: exit water tank
- 7: water collecting pipes
- 8: filtration system
- 9: particulate-filter unit
- 10: bio-filter unit
- 11: carbon-filter unit
- 12: tap water unit
- 13: reverse osmosis unit
- 14: waste water unit
- 15: pump reservoir
- 16: pump
- 17: UV-sterilization unit
- 18: fine-filter unit

- A, B, C, ...N: sampling or monitoring point

## Claims

1. System for health monitoring of aquatic species in aquacultures comprising a plurality of containments for aquatic species, wherein a sample of used water from at least one containment is supplied from at least one sample point to at least one sentinel containment housing sentinel aquatic species as bio-indicators for the detection of infectious particles and/or chemical factors and/or physical factors in said supplied water sample.

2. System according to claim 1,
**characterized by**
at least one quality monitoring device for an additionally examination of the used water supplied from the at least one sample point.

3. System according to claim 1 or 2, wherein said containments are aquaria, tanks, basins, pools, partitions of creeks, rivers or lakes and such like.

4. System according to at least one of the preceding claims, wherein the system is a re-circulating system.

5. System according to at least one of the preceding claims,
**characterized by**
at least one fresh water reservoir for supplying fresh water via water supply pipes to said containments.

6. System according to at least one of the preceding claims,
**characterized by**
water pumps providing a constant pressure in the system and water renewal in said containments.

7. System according to at least one of the claim 4 to 6,
**characterized by**
a filtration system.

8. System according to claim 7, wherein said filtration system comprises at least a large pore-filter unit, a particulate-filter unit, a fine-filter unit, a bio-filter unit, an activated carbon-filter unit and/or an UV-sterilization unit.

9. System according to at least one the claims 4 to 8,
**characterized by**
collecting pipes for collecting and supplying the used water to said particulate-filter unit, said bio-filter unit and said activated carbon-filter unit, being placed behind each other in downstream direction.

10. System according to at least one of the claims 4 to 9,
**characterized by**
a pump reservoir to which the used water is supplied via said particulate-filter unit, said bio-filter unit and said activated carbon-filter unit and to which tap water is supplied via a reverse osmosis unit.

11. System according to at least one of the claims 4 to 10, wherein said UV-sterilization unit is placed between said pump reservoir and said fresh water reservoir.

12. System according to at least one of the claims 4 to 11, wherein said fine filter unit is placed between said UV-sterilization unit and said fresh water reservoir.

13. System according to at least one of the claims 4 to 12, wherein sample points are placed for fresh water sampling, water reservoir sampling, sentinel containment sampling, exit water sampling, pipe sampling, filter sampling, fresh tap water sampling, reverse osmosis unit sampling, pump reservoir sampling, pump sampling, UV-sterilization unit sampling and / or fine filter unit sampling.

14. System according to at least one of the preceding claims, wherein the aquatic species are fish and wherein the sentinel aquatic species are fish, which are highly susceptible for fish pathogens.

15. Method for health monitoring of aquatic species in aquacultures of a system comprising a plurality of containments for aquatic species, wherein a sample of used water from at least one containment is supplied from at least one sample point to at least one sentinel containment housing sentinel aquatic species as bio-indicators for the detection of infectious particles in said supplied water sample.
